# EUROPÄISCHE PATENTSCHRIFT

(11) **EP 0 572 994 B1**
(45) Veröffentlichungstag und Bekanntmachung des Hinweises auf die Patenterteilung: **17.01.1996**
(21) Anmeldenummer: 93108836.3
(22) Anmeldetag: 02.06.1993
(51) Int. Cl.: C07D 229/00, C08G 18/79, C08F 222/08, C08F 226/06

(54) **Verfahren zur Herstellung von Uretdiongruppen aufweisenden Polyisocyanaten**
Process for the preparation of urethdione groups-containing polyisocyanates
Procédé de préparation de polyisocyanates contenant des groupes d'uréthdione

(30) Priorität: 05.06.1992 DE 4218540
(43) Veröffentlichungstag der Anmeldung: 08.12.1993
(73) Patentinhaber: BASF Aktiengesellschaft, D-67063 Ludwigshafen (DE)
(72) Erfinder: Bruchmann, Bernd, Dr., W-6700 Ludwigshafen (DE); Minges, Roland, Dr., W-6718 Gruenstadt (DE); Schade, Christian, Dr., W-6700 Ludwigshafen (DE)

(56) Entgegenhaltungen:
- EP-A- 0 317 744
- EP-A- 0 368 217
- EP-A- 0 417 603
- EP-A- 0 418 639
- EP-A- 0 431 331

## Beschreibung

Gegenstand der Erfindung ist ein Verfahren zur Herstellung von Uretdiongruppen aufweisenden Polyisocyanaten durch katalytische Dimerisierung von monomeren Isocyanaten.

Es ist grundsätzlich bekannt, Uretdione durch Umsetzung von Isocyanaten in Gegenwart von Katalysatoren herzustellen. Als Katalysatoren eignen sich verschiedene Substanzen, z.B. gemäß DE-A-30 05 106 Trialkylphosphine, gemäß DE-A-23 49 726 Trialkylphosphite, gemäß US-A-3 290 288 und DE-A-34 37 635 Triaminophosphine, gemäß JP-A-46/37503 Cycloamidine, gemäß GB-A-821 158 Pyridin bzw. substituierte Pyridine und gemäß DE-A-36 40 855 sowie gemäß DE-A-34 20 114 metallorganische Substanzen, z.B. Wismut- oder Antimonverbindungen.

Als besonders geeignet haben sich die Imidazole und Benzimidazole gemäß EP-A-0 417 603 erwiesen. Sie bewirken eine hohe Ausbeute und eine hohe Selektivität, insbesondere hinsichtlich der Unterdrückung der Isocyanuratbildung.

Diesen bekannten Katalysatoren ist gemeinsam, daß nach der Dimerisierung der Katalysator aus dem Reaktionsgemisch entfernt bzw. durch geeignete Maßnahmen desaktiviert werden muß. Verbleibt der Katalysator im Produkt, so kann er Reaktionen, bei denen das Dimer als Edukt eingesetzt wird, stören, indem er unerwünschte Nebenreaktionen katalysiert. Außerdem besteht die Möglichkeit, daß bei thermischer Behandlung der Isocyanate bzw. der daraus hergestellten Produkte der Katalysator ausgast oder sublimiert, was wegen der Gesundheitsschädlichkeit vieler der bekannten Katalysatoren vermieden werden muß.

Das Entfernen des Katalysators geschieht üblicherweise beim Waschen oder Umkristallisieren der Dimeren. Da das Produkt thermisch nicht belastet werden darf, kommt eine destillative Entfernung des Katalysators nicht in Betracht.

Das Waschen des Uretdions erfordert erhebliche Mengen an Lösungsmittel. Der Katalysator muß entweder aus den großen lösungsmittelmengen zurückgewonnen oder durch geeignete Chemikalien desaktiviert werden.

Bei den genannten Methoden ist der Katalysator nur schwer ohne nennenswerte Verluste wiederzugewinnen. Nach einer Desaktivierung verbleiben oftmals erhebliche Mengen der desaktivierten Verbindungen im Produkt.

Aufgabe der Erfindung war es, ein Verfahren für die Herstellung von Uretdiongruppen aufweisenden Polyisocyanaten aufzuzeigen, das mit hoher Ausbeute und Selektivität arbeitet und bei dem sich nach Beendigung der Uretdiongruppenbildung der Katalysator leicht aus dem Reaktionsgemisch entfernen und sich ohne aufwendige Aufarbeitung wiederverwenden läßt.

Es wurde überraschenderweise gefunden, daß diese Aufgabe gelöst wird, wenn man in dem Verfahren zur Herstellung von Uretdiongruppen aufweisenden Polyisocyanaten als Katalysatoren end- und/oder seitenständig Imidazolgruppen tragende Polymere einsetzt.

Gegenstand der Erfindung ist dementsprechend ein Verfahren zur Herstellung von Uretdiongruppen aufweisenden Polyisocyanaten durch Umsetzung von monomeren Isocyanaten in Gegenwart von Katalysatoren, dadurch gekennzeichnet, daß als Katalysatoren Polymere eingesetzt werden, die an der Polymerkette end- und/oder seitenständig Imidazolgruppen gebunden enthalten.

Bei den erfindungsgemäß als Katalysator einzusetzenden polymeren Verbindungen können die Imidazolgruppen direkt oder über Spacergruppen mit der Polymerkette verbunden sein; die Imidazolgruppen können unsubstituiert oder substituiert sein.

Als an die Polymerkette gebundene Imidazolgruppen kommen dabei insbesondere solche entsprechend den allgemeinen Formeln (I) und (II) in Betracht, 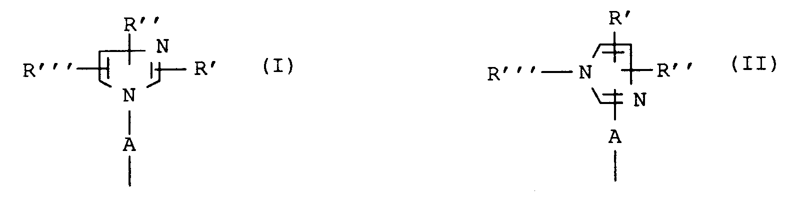
wobei
- R',R'',R''': gleich oder verschieden und unabhängig voneinander ein Wasserstoffatom, eine C₁- bis C₁₂-Alkylgruppe, eine Arylgruppe, eine C₁- bis C₁₂-Heteroalkylgruppe, eine Heteroarylgruppe, eine C₂- bis C₁₂-Alkenylgruppe, eine C₂- bis C₁₂-Alkinylgruppe, eine Ethergruppe, eine Estergruppe oder ein Halogenatom bedeuten oder auch zwei dieser Reste ringförmig, beispielsweise unter Ausbildung eines ankondensierten Benzolrings, miteinander verbunden sein können und
- A: steht für eine einfache chemische Bindung, eine C₁- bis C₁₂-Alkylengruppe, eine Arylengruppe, eine C₁- bis C₁₂-Heteroalkylengruppe, eine Heteroarylengruppe, eine C₂- bis C₁₂-Alkenylengruppe, eine C₂- bis C₁₂-Alkinylengruppe, ein Schwefelatom, eine Sulfinylgruppe oder eine Sulfonylgruppe.

Die erfindungsgemäß als Katalysatoren einzusetzenden Polymeren können durch Polymerisation von Alkenyl-, insbesondere Vinyl- oder Allylgruppen tragenden monomeren Imidazolen, allein, in Mischung miteinander oder in Mischung mit anderen copolymerisierbaren Vinyl- oder Allylmonomeren hergestellt werden.

Als Vinyl- oder Allylgruppen tragende monomere Imidazole kommen solche der allgemeinen Formeln (III) und (IV) in Betracht, 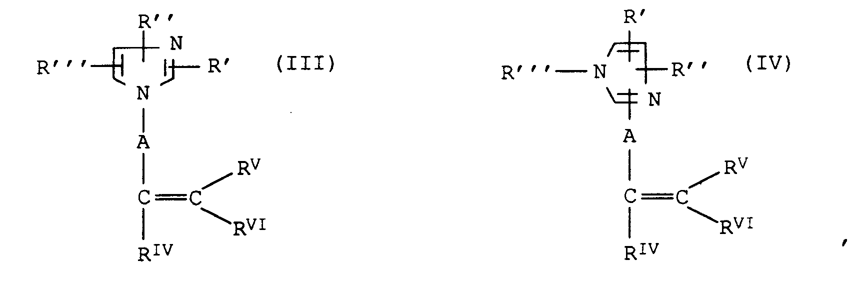
wobei
- A, R',R'',R''': die vorstehend angegebene Bedeutung haben und
- R^{IV}, R^{V} und R^{VI}: gleich oder verschieden und unabhängig voneinander ein Wasserstoffatom, eine C₁- bis C₈-, vorzugsweise C₁- bis C₄-Alkylgruppe, eine Aryl-, vorzugsweise Phenylgruppe, ein Halogen-, insbesondere Fluor- oder Chloratom, Heteroatom, insbesondere Sauerstoff und/oder Stickstoff enthaltende Gruppen, bevorzugt Ether-, Ester-, Keto- oder Säurederivat-, insbesondere Nitrilgruppen, bedeuten.

Beispiele für monomere Imidazole sind z.B. 1-Vinylimidazol, 4-Vinylimidazol, 2-Methyl-1-vinylimidazol, 4-Methyl-1-vinylimidazol, 5-Methyl-1-vinylimidazol, 2-Ethyl-1-vinylimidazol, 2-Propyl-1-vinylimidazol, 2-Isopropyl-1-vinylimidazol, 2-Phenyl-1-vinylimidazol, 1-Vinyl-4,5-benzimidazol, 1-Allylimidazol oder 1-Allyl-2-methylimidazol. Bevorzugt werden 1-Vinylimidazol, 2-Methyl-1-vinylimidazol, 1-Allylimidazol und 1-Allyl-2-methylimidazol.

Die monomeren Alkenylgruppen tragenden Imidazole können auch in Mischung mit weiteren Vinyl- oder Allylgruppen tragenden Monomeren polymerisiert werden. Derartige Comonomere sind z.B. aromatische Monomere wie Styrol, (Meth-)acrylsäurealkylester, insbesondere mit 1-20 C-Atomen in der Alkylgruppe, wie z.B. Stearylacrylat, (Meth-)acrylsäureester mit Dialkylaminogruppen in der Ester-Seitenkette, wie z.B. Diethylaminoethylacrylat, Alkylvinylester mit 1-18 C-Atomen in der Alkylgruppe, N-Vinylamide wie N-Vinylpyrrolidon und N-Vinylcaprolactam oder N-Alkyl- und N,N-Dialkyl-acrylamide. Besonders bevorzugt sind aromatische Comonomere wie z.B. Styrol.

Die erfindungsgemäß als Katalysator einzusetzenden Polymeren werden vorteilhafterweise unter Verwendung einer vernetzenden Komponente hergestellt. Dazu eignen sich als Comonomere ethylenisch mehrfach ungesättigte Monomere wie z.B. Divinylbenzol, Divinylethylenharnstoff, (Meth-)Acrylsäureallylester oder (Meth-)Acrylsäureester organischer Verbindungen mit mindestens zwei OH-Gruppen im Molekül, wie beispielsweise (Meth-)Acrylsäureester von Pentaerythrit, Trimethylolpropan oder Bisphenol-A.

Für den erfindungsgemäßen Einsatz als Katalysator sollte der Anteil an Imidazol tragenden Monomereinheiten im Polymeren in aller Regel mindestens 0,5 Gew.-% bezogen auf das Polymere, betragen, bevorzugt ist ein Bereich von 10 bis 90 Gew.-%.

Die Polymeren können nach zahlreichen, im Prinzip literaturbekannten Verfahren hergestellt werden. Zweckmäßigerweise werden die Monomere unter Verwendung radikalbildender Initiatoren bei höheren Temperaturen in an sich üblicher Weise miteinander polymerisiert. Geeignet sind insbesondere die Methoden der Suspensionspolymerisation, der umgekehrten Suspensionspolymerisation oder der Fällungspolymerisation.

Suspensionspolymere werden beispielsweise erhalten, indem die Monomere in einer wäßrigen Alkalisalz-Lösung suspendiert und umgesetzt werden. Die Eigenschaften der erhaltenen Polymeren können durch Zugabe organischer Fällungs- oder Quellungsmittel, sogenannter Porenbildner wie z.B. Essigsäureethylester oder n-Butanol, und geeigneter Schutzkolloide oder Emulgatoren zum Polymerisationsansatz in gewünschter Weise beeinflußt werden.

Bei der Methode der umgekehrten Suspensionspolymerisation werden die Monomeren in Wasser gelöst und diese Phase in einem inerten organischen Solvens wie beispielsweise Cyclohexan suspendiert und polymerisiert. Dem System werden günstig Schutzkolloide oder Emulgatoren zugegeben. Nach Beendigung der Reaktion kann das Wasser z.B. durch azeotrope Destillation entfernt und das Polymere durch Filtration isoliert werden.

Eine weitere geeignete Darstellungsmethode beruht auf der Verwendung eines Lösungsmittels, das die Monomeren, nicht jedoch das Polymer zu lösen vermag. Geeignete derartige Lösungsmittelsysteme sind Ketone mit 3-6 C-Atomen, Alkylester mit 3-12 C-Atomen, Alkohole mit 4-10 C-Atomen, Dimethylformamid, Dimethylacetamid, N-Methylpyrrolidon, t-Butylmethylether, C₁-C₄-Mono- und Dialkylether von Mono- und Oligoethylenglykolen, aromatische Lösungsmittel wie Benzol, Xylol oder Toluol, halogenierte Kohlenwasserstoffe wie 1,1,1-Trichlorethan oder Methylenchlorid, C₅-C₈-Kohlenwasserstoffe oder Mischungen dieser Lösungsmittel.

Die Polymerisation wird bevorzugt in Gegenwart einer Radikale bildenden Verbindung durchgeführt. Geeignete Initiatoren sind z.B. Wasserstoffperoxyd, anorganische Persulfate oder organische Verbindungen vom Peroxid- oder Azotyp. Das Molekulargewicht der Polymeren kann gegebenenfalls durch Zugabe von Reglern in der Reaktionsmischung erniedrigt werden.

Die nach diesen Methoden dargestellten Polymeren lassen sich im allgemeinen gut, z.B. durch Filtration, isolieren und durch weitere geeignete Schritte reinigen und trocknen. Um bei der erfindungsgemäßen Verwendung der Polymeren als Katalysatoren eine größere aktive Oberfläche zu erhalten, ist es oft sinnvoll, die Polymeren vor ihrem Einsatz in dem erfindungsgemäßen Verfahren, z.B. durch Vermahlen bis auf Teilchengrößen von 10 bis 250 µm, bevorzugt 20 bis 150 µm, zu zerkleinern.

Um bestimmte Eigenschaften der Imidazolgruppen enthaltenden Polymeren, z.B. Temperatur- und Lösungsmittelbeständigkeit oder Abriebverhalten, positiv zu beeinflussen, können diese mit handelsüblichen Polymeren, z.B. Polyethylen, Polypropylen, Polyvinylchlorid, Polytetrafluorethylen, Polyetherketon, in Mischung verwendet werden. Die Herstellung dieser Blends erfolgt in an sich bekannter Weise.

Eine weitere Möglichkeit der Herstellung der erfindungsgemäßen Imidazolgruppen enthaltenden Polymeren besteht in der Umsetzung von reaktive Gruppen enthaltenden Imidazolen mit Polymeren, an deren Ketten end- und/oder seitenständig mit den reaktiven Gruppen der Imidazole reaktionsfähige Gruppen enthalten sind.

Die end- und/oder seitenständig Imidazolgruppen tragenden Polymeren werden erfindungsgemäß als Katalysatoren bei der Umsetzung monomerer Isocyanate zur Herstellung von Uretdiongruppen enthaltenden Polyisocyanaten eingesetzt.

Die Dimerisierung der monomeren Isocyanate erfolgt in Substanz oder in einem aprotischen Lösungsmittel. Die Katalysatorkonzentration sollte mindestens 0,001 Gew.-%, bezogen auf das monomere Isocyanat, betragen und liegt üblicherweise im Bereich von 0,01 bis 5 Gew.-%, bezogen auf das eingesetzte monomere Isocyanat.

Als Isocyanate können aromatische Isocyanate, z.B. Phenylisocyanat, Tolylisocyanat, 1,5-Naphthalindiisocyanat, 1,4-Phenylendiisocyanat, 2,4-Toluylendiisocyanat (2,4-TDI), 2,6-Toluylendiisocyanat (2,6-TDI), 4,4'-Diphenylmethandiisocyanat (4,4'-MDI), 2,4'-Diphenylmethandiisocyanat (2,4'-MDI) einzeln oder als Gemische untereinander, zum Einsatz kommen.

Als Lösungsmittel werden aprotische organische Lösungsmittel eingesetzt, insbesondere Benzol, Toluol, Aceton, Methylethylketon, Chloroform, Essigsäurealkylester und n-, iso- oder Cycloalkane oder Chlorbenzol.

Die Dimerisierung setzt nach der Zugabe des Katalysators zu dem in Substanz oder in Lösung vorliegenden Isocyanat meist schnell ein. Nach erfolgter Dimerisierung kann erfindungsgemäß der polymere Katalysator sehr einfach aus dem Reaktionsgemisch entfernt werden, z.B. indem der Katalysator bei beginnender Trübung des Reaktionsgemisches rasch abfiltriert wird. Danach kann das Uretdiongruppen enthaltende Polyisocyanat durch Abkühlung der Reaktionslösung auskristallisiert werden. Die Abtrennung erfolgt z.B. durch Filtration. Das abgetrennte Produkt wird mit einem aprotischen organischen Lösungsmittel gewaschen und, zur Vermeidung thermischer Schädigungen, unter Vakuum getrocknet. Der abgetrennte Katalysator kann, ohne weitere Behandlung, für den nächsten Reaktionsansatz wiederverwendet werden.

Es ist auch möglich, die Dimerisierung der Isocyanate kontinuierlich zu betreiben, indem der polymere Katalysator als Festbett eingesetzt und das Isocyanat in Substanz oder in Lösung darübergefahren wird.

Wenn die katalytische Aktivität des Katalysators durch Belegung mit Isocyanaten oder Reaktionsprodukten nachläßt, kann er durch Behandlung mit einem Lösungsmittel oder thermische Behandlung von den anhaftenden Produkten befreit und somit reaktiviert werden.

Das erfindungsgemäße Verfahren ist einfach und problemlos durchführbar. Die erfindungsgemäß einzusetzenden Polymerkatalysatoren zeigen dabei überraschenderweise eine sehr hohe Aktivität und Selektivität, führen zu reinen Endprodukten, ohne daß diese einer besonderen Reinigung bedürften, und sind wiederverwendbar.

Die Erfindung wird durch die nachfolgenden Beispiele näher erläutert. Die angegebenen Teile beziehen sich, sofern nicht anders vermerkt, auf das Gewicht.

### Beispiel 1

### Herstellung der polymeren Katalysatoren

Es wurden folgende Polymerkatalysatoren hergestellt (siehe auch Tabelle 1)
A) Copolymer aus 1-Vinylimidazol (1) und Styrol, 50:50, vernetzt mit Divinylethylenharnstoff (DVEH)
B) Copolymer aus 1-Vinylimidazol (1) und Bisphenol-A-diacrylat (BADA), 80:20
C) Copolymer aus 2-Methyl-1-vinylimidazol (2) und Styrol, 66:33, vernetzt mit DVEH
D) Copolymer aus 2-Methyl-1-vinylimidazol (2) und Styrol, 20:80, vernetzt mit DVEH
E) Copolymer aus 1-Allyl-2-methylimidazol (3) und BADA, 83:17,

**Tabelle 1**

| Ansätze für die Katalysatorherstellung | |
|---|---|
| Polymer | Ansatz |
| A | 200 g Essigester |
| | 75 g Imidazol 1 |
| | 75 g Styrol |
| | 7,5 g DVEH |
| | 1,5 g AIBN (Azobisisobutyronitril) |
| B | 250 g Essigester |
| | 150 g Imidazol 1 |
| | 30 g BADA |
| | 4 g AIBN |
| C | 200 g n-Butanol |
| | 100 g Imidazol 2 |
| | 50 g Styrol |
| | 7,5 g DVEH |
| | 1,5 g AIBN |
| D | 200 g n-Butanol |
| | 30 g Imidazol 2 |
| | 120 g Styrol |
| | 7,5 g DVEH |
| | 1,5 g AIBN |
| E | 250 g Essigester |
| | 150 g Imidazol 3 |
| | 30 g BADA |
| | 4 g AIBN |

### Durchführung der Katalysatorherstellung

In ein beheizbares und mit einem Rührer versehenes Reaktionsgefäß wurde eine Lösung von 1 100 g Wasser und 200 g Natriumsulfat vorgelegt und mit Stickstoff begast. Unter Rühren wurde der jeweilige Ansatz (siehe Tabelle 1) zugegeben und auf 70°C aufgeheizt. Die Mischung wurde 22 h bei 70°C gerührt.

Das entstandene Polymerisat wurde anschließend nach Abkühlen der Reaktionsmischung über eine Filternutsche isoliert, mehrfach mit Wasser gewaschen und danach unter Vakuum bei 60°C 16 Stunden lang getrocknet. Anschließend wurde es auf eine Teilchengröße von < 250 µm vermahlen.

### Beispiel 2

### Dimerisierung der Isocyanate

### Allgemeine Reaktionsbedingungen:

1 000 g Diisocyanat, 5 g Polymerkatalysator und gegebenenfalls 1 000 g Lösungsmittel, jeweils wie in Tabelle 2 angegeben, wurden auf 40°C erwärmt und 2 Stunden gerührt. Danach wurde der Katalysator abgesaugt. Beim Abkühlen auf 20°C kristallisierten das Dimer als Feststoff aus. Es wurde nach 12 Stunden abgesaugt, mit Toluol oder Methylethylketon (MEK) gewaschen und im Vakuum bei 60°C getrocknet. Die Uretdione enthielten noch Spuren von anhaftenden Monomeren. Isocyanurate, die bei der Verwendung der Uretdione in Polyurethansystemen eine meist unerwünschte Vernetzung verursachen, lagen unterhalb der Nachweisgrenze (ca. 1 %).

**Tabelle 2**

| Reaktionsdaten der Dimerisierung und Produktdaten der Dimere | | | | |
|---|---|---|---|---|
| Isocyanat | Lösungsmittel | Katalysator | Ausbeute [%] | Schmelzpunkt [°C] |
| 2,4-TDI | Toluol | A | 15 | 155-158 |
| 2,4-TDI | - | B | 12 | 155-158 |
| 2,4-TDI | MEK | C | 30 | 157-159 |
| 2,4-TDI und 2,6-TDI im Verhältnis 80:20 | Toluol | D | 10 | 156-158 |
| 2,4-TDI | - | E | 35 | 156 |
| 2,4-TDI | MEK | E | 45 | 156-158 |
| 2,4-TDI | Toluol | E | 40 | 156 |
| 4,4'-MDI | MEK | E | 42 | 256-275 |

### Beispiel 3

### Wiederverwendbarkeit der Katalysatorsysteme

1 000 g 2,4-TDI, 10 g Katalysator E und 1 000 g Methylethylketon wurden bei 40°C 1 Stunde gerührt. Der Katalysator wurde abgesaugt (Filtrat 1) und erneut in eine Mischung aus 1 000 g 2,4-TDI und 1 000 g Methylethylketon bei 40°C eingesetzt. Nach 1 Stunde wurde der Katalysator wieder abgesaugt (Filtrat 2), mit Toluol und danach mit Methylethylketon ausgekocht und nochmals mit 1 000 g 2,4-TDI und 1 000 g Methylethylketon eingesetzt. Nach dem Absaugen des Katalysators erhielt man Filtrat 3. Aus den Filtraten kristallisierte beim Abkühlen auf 20°C das TDI-Dimer aus. Dieses wurde nach 12 Stunden abgesaugt und wie in Beispiel 2 weiterbehandelt. Die Schmelzpunkte der Dimere lagen bei 156 bis 158°C.

Es wurde folgende Ausbeuten erzielt:
Filtrat 1: 520 g (52 %)
Filtrat 2: 280 g (28 %)
Filtrat 3: 390 g (39 %)

## Patentansprüche

1. Verfahren zur Herstellung von Uretdiongruppen aufweisenden Polyisocyanaten durch Dimerisierung von monomeren Isocyanaten in Gegenwart von Katalysatoren, dadurch gekennzeichnet, daß als Katalysatoren polymere Verbindungen, an deren Polymerketten end- und/oder seitenständig Imidazolgruppen gebunden sind, eingesetzt werden.

2. Verfahren nach Anspruch 1, dadurch gekennzeichnet, daß die Imidazolgruppen unsubstituiert oder substituiert sein können.

3. Verfahren nach einem der Ansprüche 1 oder 2, dadurch gekennzeichnet, daß die als Katalysator eingesetzten Imidazolgruppen enthaltenden Polymeren durch Polymerisation alkenylgruppenhaltiger Imidazole allein oder zusammen mit Vinylmonomeren hergestellt worden sind.

4. Verfahren nach einem der Ansprüche 1 bis 3, dadurch gekennzeichnet, daß die als Katalysator eingesetzten Imidazolgruppen enthaltenden Polymeren in Mischung mit herkömmlichen Polymeren als Katalysatoren eingesetzt werden können.

## Claims

1. A process for the preparation of polyisocyanates containing uretdione groups by dimerizing monomeric isocyanates in the presence of catalysts, wherein the catalysts employed are polymeric compounds to whose polymer chains imidazole groups are bonded terminally and/or laterally.

2. A process as claimed in claim 1, wherein the imidazole groups may be unsubstituted or substituted.

3. A process as claimed in claim 1 or 2, wherein the imidazole group-containing polymers employed as catalyst have been prepared by polymerizing alkenyl group-containing imidazoles, alone or together with vinyl monomers.

4. A process as claimed in claim 1 or 2 or 3, wherein the imidazole group-containing polymers employed as catalyst can be employed as a mixture with conventional polymers.

## Revendications

1. Procédé pour la préparation de polyisocyanates présentant des groupes urétdione par dimérisation d'isocyanates monomères en présence de catalyseurs, caractérisé en ce qu'on emploie, comme catalyseurs, des composés polymères, sur les chaînes polymères desquels sont fixés des groupes imidazole en position terminale et/ou latérale.

2. Procédé selon la revendication 1, caractérise, en ce que les groupes imidazole peuvent être substitués ou non substitués.

3. Procédé selon l'une des revendications 1 ou 2, caractérisé en ce que les polymères contenant des groupes imidazole employés comme catalyseur ont été préparés par polymérisation d'imidazoles contenant des groupes alcényle, seuls ou conjointement avec des monomères vinyliques.

4. Procédé selon l'une quelconque des revendications 1 à 3, caractérisé en ce que les polymères contenant des groupes imidazole employés comme catalyseur sont utilisés en mélange avec des polymères classiques comme catalyseurs.
